(19) Europäisches Patentamt
European Patent Office
Office européen des brevets

(11) Publication number: **0 146 922**
**B1**

(12) **EUROPEAN PATENT SPECIFICATION**

(45) Date of publication of patent specification: **07.09.88**

(51) Int. Cl.$^4$: **C 07 D 311/36**, A 61 K 31/35

(21) Application number: **84115840.5**

(22) Date of filing: **19.12.84**

(54) **Benzopyran-4-on derivatives and their production.**

(30) Priority: **21.12.83 JP 242780/83**

(43) Date of publication of application:
**03.07.85 Bulletin 85/27**

(45) Publication of the grant of the patent:
**07.09.88 Bulletin 88/36**

(84) Designated Contracting States:
**BE CH DE FR GB IT LI NL SE**

(56) References cited:
**EP-A-0 146 921**
**DE-B-1 210 882**
**FR-A-2 190 411**

**CHEMICAL ABSTRACTS, vol. 78, 1973, page 345, no. 4125c, Columbus, Ohio, US; & JP - A - 72 32 074 (SAKAI CHEMICAL INDUSTRY CO., LTD.) 17-08-1972**

(73) Proprietor: **Takeda Chemical Industries, Ltd.**
**27, Doshomachi 2-chome Higashi-ku**
**Osaka-shi Osaka, 541 (JP)**

(72) Inventor: **Yamazaki, Iwao**
**9-21, Hibarigaokayamata 1-chome**
**Takarazuka Hyogo 665 (JP)**
Inventor: **Sawa, Yoichi**
**19-11, Sengokuhigashi-machi**
**Kadoma Osaka 571 (JP)**

(74) Representative: **von Kreisler, Alek, Dipl.-Chem.**
**Deichmannhaus am Hauptbahnhof**
**D-5000 Köln 1 (DE)**

**EP 0 146 922 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

On page 1 of FR—A—2 190 411 a group of compounds comprising 7-(1-hydroxycarbonylethoxy)-3-(4-hydroxyphenyl)chromone — which is, however, not specifically disclosed — are reported to have a vascular protective effect. The compound 7-ethoxycarbonyl(methoxy-3-phenylchromone is disclosed in Chemical Abstracts *78,* 4125c (1973) as an anticonvulsant and in DE—A—1 210 882 as a vasodilator.

The Applicant's conflicting EP—A—0 146 921 of the same effective date has been abandoned.

This invention relates to novel benzopyran-4-on derivatives and their production. More particularly, it relates to 3-phenyl-4H-1-benzopyran-4-one derivatives (hereinafter referred to as compounds [I]) of the formula

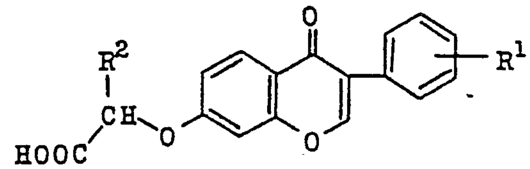

〔 I 〕

wherein $R^1$ is hydrogen or hydroxy and $R^2$ is lower alkyl and production thereof.

The compounds [I] are produced, for example, by reacting, in accordance with the reaction scheme given below, a hydroxy compound [II] with a 2-haloalkanecarboxylic acid or an ester thereof [III], if necessary followed by hydrolysis.

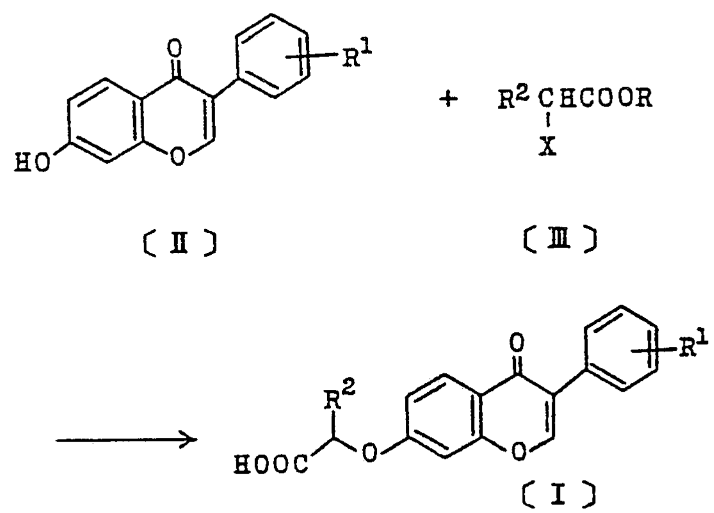

〔 II 〕 + 〔 III 〕

〔 I 〕

In the above formulas, X is halogen, $R^1$ is hydrogen or hydroxy, $R^2$ is lower alkyl and R is hydrogen or lower alkyl.

The starting compound [II] is produced, for example, by the method of E. M. Gaydou et al. (Bull. Soc. Chim. Fr., *1978,* II—43) or the method of S. A. Kagal et al. (Tetrahedron Letters, *1962,* 593). The starting compound [III] is, for example, 2 - chloropropionic acid, 2 - bromobutyric acid, 2 - iodovaleric acid, 2 - bromocaproic acid, methyl 2 - chloropropionate, methyl 2 - bromobutyrate, ethyl 2 - chloropropionate, ethyl 2 - bromovalerate, propyl 2 - chloropropionate, propyl 2 - bromocaproate, butyl 2 - chloropropionate, butyl 2 - bromopropionate or methyl 2 - idoheptanhoate.

Generally, the reaction in accordance with the present invention is advantageously carried out in the presence of an inert solvent. As such solvent, there may be used any solvent which will not interfere with said reaction. Such solvent may suitable be selected, for instance, from among alcohols (e.g. methanol, ethanol, propanol, isopropyl alcohol, butanol), ketones (e.g. acetone, methyl, isobutyl ketone, cyclohexanone), ethers (e.g. tetrahydrofuran, dioxane, diethyl ether, isopropyl ether), halogenated hydrocarbons (e.g. dichloromethane, dichloroethane, chloroform), petroleum ether, toluene, xylene, dimethylformamide, pyridine, aldehyde collidine, water, and mixed solvents composed of two or more of these.

The above condensation reaction is advantageously carried out in the presence of a deacidifying agent, such as an alkali metal hydrogen carbonate (e.g. sodium hydrogen carbonate, potassium hydrogen carbonate), an alkali metal hydroxide (e.g. sodium hydroxide, potassium hydroxide), an alkali metal carbonate (e.g. sodium carbonate, potassium carbonate), sodium amide, sodium hydride, an alkali metal (e.g. sodium, potassium), a sodium alocholate (e.g. sodium methylate, sodium ethylate), or an organic base (e.g. pyridine, aldehyde collidine, dimethylaniline, triethylamine).

The hydrolysis of a 7-hydroxy-α-lower alkyl-acetic acid ester-benzopyran-4-one derivative obtained by

using a 2-haloalkanecarboxylic acid ester as the starting compound [III] is carried out in the conventional manner. Thus, the hydrolysis is conducted in the presence of a catalyst usable in generally known hydrolysis reactions. Said catalyst is, for instance, an inorganic acid (e.g. sulfuric acid, hydrochloric acid, hydrobromic acid), an organic acid [e.g. toluenesulfonic acid, benzenesulfonic acid, strongly acidic ion exchange resin (e.g. Amberlite IR—120)], or a mixture thereof.

Referring to the above formula [III], the halogen represented by X includes chlorine, bromine, iodine, fluorine, etc., whereas the lower alkyl represented by R includes those containing 1 to 4 carbon atoms, such as methyl, ethyl, propyl, isopropyl, butyl, isobutyl, secondary butyl and tertiary butyl.

The reaction temperature, solvent and other reaction conditions may be selected in an adequate manner depending on each specific case.

The thus-produced compounds [I] can be isolated from the reaction mixture and purified by conventional methods of treatment (e.g. extraction, recrystallization, chromatography).

The compounds [I] directly act on the bone and thereby produce excellent bone resorption inhibiting activity. Therefore, they are suited for use as osteoporosis-treating agents.

Osteoporosis is a disease condition or illness wherein the quantitative loss of bones has progressed beyond a certain limit to thereby present some symptoms or risk manifestations. Among its main clinical manifestations are kyphosis, low back pain, and fractures of femoral neck, lower end of the radius, ribs, upper end of the humerus, etc. While the pothogenic factors are variegated, including endocrine disorder and nutritional disorder. There are therapeutic agents such as estrogen, calcitonin, vitamin D and calcium preparations. However, these drugs were either limited in applications or only indefinitely effective, so that none was found to be fully satisfactory. The compounds [I] provide a remedy or more general nature against osteoporosis.

Test Example 1

Bone resorption inhibiting activity of 7-(1-hydrocarbonylethyl)oxy-3-phenyl-4H-1-benzopyran-4-one (hereinafter referred to as compound A) in rat fetal long bone culture

Determination of bone resorption was performed by the method of Raisz [J. Clin. Invest. *44,* 103—116 (1965)]. Thus, a Sprague-Dawley rat on the 19th day of pregnancy was subcutaneously injected with 50 μCi of $^{45}Ca$ (isotope of calcium, $CaCl_2$ solution), and was laparotomized on the following day. The embryos were aseptically taken out, the forelimbs (radius and ulna) were cut off from the trunk under a binocular dissecting microscope, and the connective tissue and cartilage were removed as much as possible to prepare bone samples. Each bone sample was preincubated at 37°C for 24 hours in 0.6 ml of the medium containing 2 mg/ml of bovine serum albumin in $BGJ_b$ medium (Fitton-Jackson modification) [GIBCO Laboratories, Grand Island, NY 14072 U.S.A.]. Then, the sample was further incubated for 3 days in the same medium as above, in which 10 μg/ml of compound A had been incorporated. Then, the radioactivity of $^{45}Ca$ in the medium and that of $^{45}Ca$ in the bone were measured and the percentage (%) of $^{45}Ca$ released from the bone into the medium was calculated by the following formula.

Percentage (%) of $^{45}Ca$ released from bone into medium

$$= \frac{\text{Count of } ^{45}\text{Ca in medium}}{\text{Count of } ^{45}\text{Ca in medium} + \text{Count of } ^{45}\text{Ca in bone}} \times 100$$

As control, the bones of the embryos from the same litter were similarly incubated in the absence of compound A for 3 days. Then mean ± standard deviation for the six bones per group are shown in Table 1. It is apparent that compound A suppressed bone resorption.

TABLE 1

| | Concentration of compound A | $^{45}Ca$ (%) released |
|---|---|---|
| Control group | 0 | 23.6 ± 3.9 |
| Test group | 10 μg/ml | 18.5 ± 2.2* |

*: A significant difference from the control group ($p<0.05$)

Test Example 2

Potentiating effect of 7-(1-hydroxycarbonylethyl)oxy-3-(4-hydroxyphenyl)-4H-1-benzopyran-4-one (hereinafter referred to as compound B) on the bone resorption inhibiting action of calcinin in rat fetal long bone culture

The bone samples prepared in the same manner as Test Example 1 were preincubated for 24 hours in the same medium as that prepared in Test Example 1 which contains bovine serum albumin in $BGJ_b$ medium (Fitton-Jackson modification).

Then, in the concomitant presence of PTH (parathyroid hormone, a bone resorption stimulant), calcitonin and compound B, the samples were further incubated for 3 days and the percentage of $^{45}Ca$ released into the medium was calculated by means of the same formula as that in Test Example 1. The results are shown in Table 2. As control experiments, the same determination was made for a control I group using the medium supplemented with PTH alone and a control II group using the medium supplemented with PTH and calcitonin. It is apparent from Table 2 that compound B potentiated the inhibitory action of calcitonin on PTH-stimulated bone resorption.

TABLE 2

| | Concentration of calcitonin | Concentration of compound B | $^{45}Ca$ (%) released |
|---|---|---|---|
| Control I group | 0 | 0 | 39.7 ± 1.5 |
| Control II group | 3 mU/ml | 0 | 35.9 ± 4.9 |
| Test group | 3 mU/ml | 25 μg/ml | 31.2 ± 3.4 |

Test Example 3
Acute toxicity

Five-week-old ICR mice and 5-week old Sprague-Dawley rats were used in groups of 10 males and 10 females, and suspensions of compound A or compound B in olive oil were administered orally [2,500, 5,000 and 10,000 mg/kg of each compound] or subcutaneously (1,250, 2,500 and 5,000 mg/kg]. The animals were kept under observation for 14 days. None of the groups showed deaths, nor toxic symptons, which might be attributable to compound A or B, with the result that $LD_{50}$ could not be calculated.

When the compounds [I] are used for the treatment of osteoporosis, it is orally administered in a usual daily dose of about 10 to 500 mg for an adult human, in such dosage forms as tablets, powders, granules, capsules, liquids, etc. or parenterally administered as, for example, injections at a dose level of about 1 to 100 mg per injection for an adult human.

Reference Example 1 Tablet

| | | |
|---|---|---|
| I) | 7-(1-Hydroxycarbonylethyl)-oxy-3-phenyl-4H-1-benzopyran-4-one | 200 g |
| II) | Lactose | 15 g |
| III) | Starch | 45 g |
| IV) | Carboxymethylcellulose calcium | 10 g |
| V) | Magnesium stearate | 1 g |

The above components I) through V) were admixed to prepare 1000 uncoated tablets with diameter of 8.5 mm.

Reference Example 2 Capsules

| | | |
|---|---|---|
| I) | 7-(1-Hydroxycarbonylethyl)-oxy-3-(4-hydroxyphenyl)-4H-1-benzopyran-4-one | 200 g |
| II) | Lactose | 40 g |
| III) | Starch | 50 g |
| IV) | Hydroxypropylcellulose | 7 g |
| V) | Magnesium stearate | 3 g |

The above components I) through V) were admixed and filled into 1000 No. 1 capsules.

Example 1—1

A mixture of 40 g of 7-hydroxy-3-phenyl-4H-1-benzopyran-4-one, 46 g of ethyl 2-bromopropionate, 250 ml of dimethylformamide and 46.5 g of potassium carbonate was stirred well with heating at 80°C for 2 hours and poured into 270 ml of ice water. The resulting crystalline precipitate was collected by filtration

and recrystallized from ethyl acetate-ethanol to give 103 g of 7 - (1 - ethoxycarbonylethyl)oxy - 3 - phenyl - 4H - 1 - benzopyran - 4 - one as white crystals.

m.p. 165—166°C.

Elemental analysis

| | | |
|---|---|---|
| Calcd. for $C_{20}H_{18}O_5$: | C, 71.00; | H, 5.36 |
| Found: | C, 71.19; | H, 5.41 |

Example 1—2

A mixture of 20 g of 7-(1-ethoxycarbonylethyl)oxy-3-phenyl-4H-1-benzopyran-4-one, 200 ml of dioxane, 70 ml of hydrochloric acid and 150 ml of water was refluxed for 4 hours and poured into ice water. The resulting crystalline precipitate was collected by filtration and recrystallized from ethanol to give 17.1 g of 7 - (1 - hydroxycarbonylethyl)oxy - 3 - phenyl - 4H - 1 - benzopyran - 4 - one as white crystals.

m.p. 223°C.

Elemental analysis

| | | |
|---|---|---|
| Calcd. for $C_{18}H_{14}O_5$: | C, 69.67; | H, 4.55 |
| Found: | C, 69.68; | H, 4.52 |

Example 2—1

A mixture of 4.0 g of 7-hydroxy-3-(4-hydroxyphenyl)-4H-1-benzopyran-4-one, 3.42 g of ethyl 2-bromopropionate, 60 ml of dimethylformamide and 4.3 g of potassium carbonate was stirred well with heating at 80°C for 2 hours, poured into ice water and extracted with ethyl acetate. The extract was washed with water and concentrated and the residue was recrystallized from dichloroethane to give 3.0 g of 7 - (1 - ethoxycarbonylethyl)oxy - 3 - (4 - hydroxyphenyl) - 4H - 1 - benzopyran - 4 - one as white crystals.

m.p. 172—173°C.

Elemental analysis

| | | |
|---|---|---|
| Calcd. for $C_{20}H_{18}O_6$: | C, 67.79; | H, 5.12 |
| Found: | C, 67.72; | H, 5.18 |

In the same manner as above, the following compounds were obtained.

7-(1-Ethoxycarbonylethyl)oxy-3-(3-hydroxyphenyl)-4H-1-benzopyran-4-one.

m.p. 193—194°C.

Elemental analysis

| | | |
|---|---|---|
| Calcd. for $C_{20}H_{18}O_6$: | C, 67.79; | H, 5.12 |
| Found: | C, 67.61; | H, 5.28 |

7-(1-Ethoxycarbonylethyl)oxy-3-(2-hydroxyphenyl)-4H-1-benzopyran-4-one.

m.p. 154—156°C.

NMR (DMSO-$d_6$) δ: 1.25 (3H, t, $CH_3$), 1.60 (3H, d, $CH_3$), 4.20 (2H, q, $CH_2$), 5.25 (1H, q, CH), 6.8—7.4 (6H, m, aromatic H), 8.05 (1H, d, $C_5$—H), 8.25 (1H, s, $C_2$—H), 9.30 (1H, bs, OH)

Example 2—2

A mixture of 3.0 g of 7 - (1 - ethoxycarbonylethyl)oxy - 3 - (3 - hydroxyphenyl) - 4H - 1 - benzopyran - 4 - one, 90 ml of dioxane, 120 ml of water and 15 ml of hydrochloric acid was refluxed for 2.5 hours, poured into ice water and extracted with ethyl acetate. The extract was washed with water and concentrated and the residue was recrystallized from ethyl acetate-dichloroethane to give 2.5 g of 7 - (1 - hydroxycarbonylethyl)oxy - 3 - (3 - hydroxyphenyl) - 4H - 1 - benzopyran - 4 - one as white crystals.

m.p. 218—219°C.

Elemental analysis

| | | |
|---|---|---|
| Calcd. for $C_{18}H_{14}O_6 \cdot 1/4H_2O$: | C, 65.34; | H, 4.72 |
| Found: | C, 65.31; | H, 4.54 |

Using 7 - (1 - ethoxycarbonylethyl)oxy - 3 - (4 - hydroxyphenyl) - 4H - 1 - benzopyran - 4 - one and following the same procedure as above, 7 - (1 - hydroxycarbonylethyl)oxy - 3 - (4 - hydroxyphenyl) - 4H - 1 - benzopyran - 4 - one was obtained as white crystals.

m.p. 201—202°C

NMR (DMSO-$d_6$) δ: 1.57 (3H, d, $CH_3$), 5.07 (1H, q, CH), 6.83 (2H, d, aromatic H), 6.95—7.20 (2H, m, $C_6$ & $C_8$—H), 8.04 (1H, d, $C_5$—H), 8.31 (1H, s, $C_2$—H)

Using 7 - (1 - ethoxycarbonylethyl)oxy - 3 - (2 - hydroxyphenyl) - 4H - 1 - benzopyran - 4 - one and following the same procedure as above, the following compound was obtained.

7 - (1 - Hydroxycarbonylethyl)oxy - 3 - (2 - hydroxyphenyl) - 4H - 1 - benzopyran - 4 - one.

m.p. 180—182°C.

NMR (DMSO-$d_6$) δ: 1.55 (3H, d, $CH_3$), 5.10 (1H, q, CH), 6.6—7.4 (6H, m, aromatic H), 8.05 (1H, d, $C_5$—H), 8.20 (1H, s, $C_2$—H), 9.3 (1H, bs, OH)

**Claims**

1. A compound of the formula

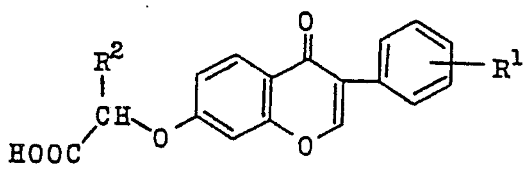

wherein $R^1$ is hydrogen or hydroxy and $R^2$ is $C_1$—$C_4$ alkyl.

2. The compound according to claim 1, wherein $R^2$ is methyl.

3. The compound according to claim 1, which is 7 - (1 - hydroxycarbonylethyl)oxy - 3 - phenyl - 4H - 1 - benzopyran - 4 - one.

4. The compound according to claim 1, which is 7 - (1 - hydroxycarbonylethyl)oxy - 3 - (4 - hydroxyphenyl) - 4H - 1 - benzopyran - 4 - one.

5. A method for producing a compound of the formula

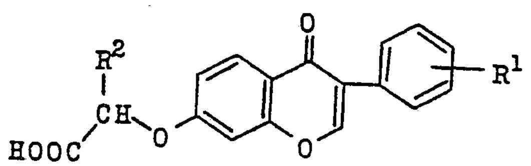

( I )

wherein $R^1$ is hydrogen or hydroxy and $R^2$ is $C_1$—$C_4$ alkyl, which comprises reacting a compound of the formula

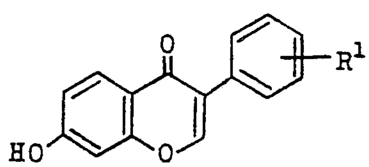

(II)

wherein $R^1$ is a defined hereinbefore, with a compound of the formula

$$R^2\text{—}\underset{\underset{X}{|}}{C}HCOOR \quad \text{(III)}$$

wherein X is halogen, R is hydrogen or lower alkyl and $R^2$ is as defined hereinbefore, if necessary followed by hydrolysis.

6. The method according to claim 5, wherein the product is produced by reacting the compound of the formula (II) with the compound of the formula (III) wherein R is lower alkyl, and hydrolyzing.

7. The method according to claim 5, wherein the product is the compound of the formula (I) wherein $R^2$ is methyl.

8. The method according to claim 5, wherein the product is 7 - (1 - hydroxycarbonylethyl)oxy - 3 - phenyl - 4H - 1 - benzopyran - 4 - one.

9. The method according to claim 5, wherein the product is 7 - (1 - hydroxycarbonylethyl)oxy - 3 - (4 - hydroxyphenyl) - 4H - 1 - benzopyran - 4 - one.

**Patentansprüche**

1. Verbindung der Formel

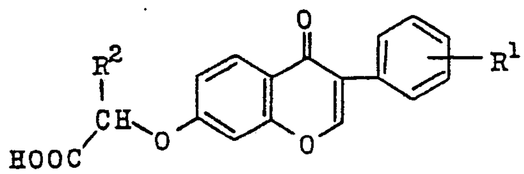

in der $R^1$ Wasserstoff oder Hydroxy ist und $R^2$ $C_1$—$C_4$-Alkyl ist.

2. Verbindung nach Anspruch 1, in der $R^2$ Methyl ist.

3. Verbindung nach Anspruch 1, die 7 - (1 - Hydroxycarbonylethyl)oxy - 3 - phenyl - 4H - 1 - benzopyran - 4 - on ist.

4. Verbindung nach Anspruch 1, die 7 - (1 - Hydroxycarbonylethyl)oxy - 3 - (4 - hydroxyphenyl) - 4H - 1 - benzopyran - 4 - on ist.

5. Verfahren zur Herstellung einer Verbindung der Formel

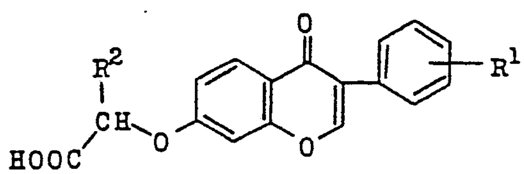

(I)

in der $R^1$ Wasserstoff oder Hydroxy ist und $R^2$ $C_1$—$C_4$-Alkyl ist, umfassend die Umsetzung einer Verbindung der Formel

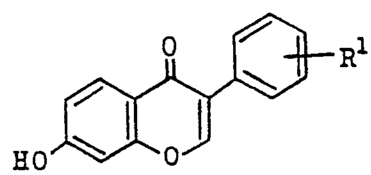

(II)

in der $R^1$ die im Vorstehenden angegebenen Bedeutungen hat, mit einer Verbindung der Formel

$R^2$—CHCOOR
|
X

(III)

in der X Halogen ist, R Wasserstoff oder niederes Alkyl ist und $R^2$ die im Vorstehenden angegebenen Bedeutungen hat, und erforderlichenfalls die nachfolgende Hydrolyse.

6. Verfahren nach Anspruch 5, worin das Produkt durch Umsetzung der Verbindung der Formel (II) mit der Verbindung der Formel (III), in der R niederes Alkyl ist, und Hydrolysieren hergestellt wird.

7. Verfahren nach Anspruch 5, worin das Produkt die Verbindung der Formel (I) ist, in der $R^2$ Methyl ist.

8. Verfahren nach Anspruch 5, worin das Produkt die Verbindung 7 - (1 - Hydroxycarbonylethyl)oxy - 3 - phenyl - 4H - 1 - benzopyran - 4 - on ist.

9. Verfahren nach Anspruch 5, worin das Produkt die Verbindung 7 - (1 - Hydroxycarbonylethyl)oxy - 3 - (4 - hydroxyphenyl) - 4H - 1 - benzopyran - 4 - on ist.

**Revendications**

1. Composé der formule

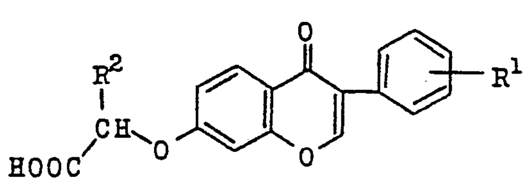

dans laquelle $R^1$ est un atome d'hydrogène ou un groupe hydroxyle, et $R^2$ est un groupe alkyle en $C_1$—$C_4$.

2. Composé conforme à la revendication 1, dans lequel $R^2$ est un groupe méthyle.

3. Composé conforme à la revendication 1, qui est la 7 - (1 - hydroxycarbonyléthyl)oxy - 3 - phényl - 4H - 1 - benzopyran - 4 - one.

4. Composé conforme à la revendication 1, qui est la 7 - (1 - hydroxycarbonyléthyl)oxy - 3 - (4 - hydroxyphényl) - 4H - 1 - benzopyran - 4 - one.

5. Procédé de production d'un composé de formule

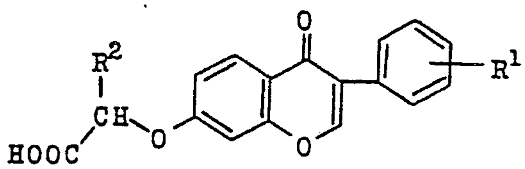

(I)

dans laquelle $R^1$ est un atome d'hydrogène ou un groupe hydroxyle et $R^2$ est un groupe alkyle en $C_1$—$C_4$, qui comporte la réaction d'un composé de formule

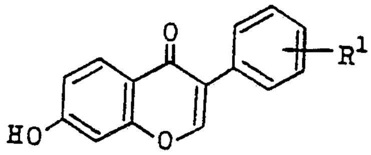

(II)

dans laquelle $R^1$ est tel que défini ci-dessus, avec un composé de formule

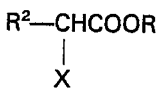

(III)

dans laquelle X est un atome d'halogène, R est un atome d'hydrogène ou un groupe alkyle inférieur et $R^2$ est tel que défini ci-dessus, sivie si nécessaire d'une hydrolyse.

6. Procédé conforme à la revendication 5, dans lequel le produit est produit par réaction du composé de formule (II) avec le composé der formule (III) dans lequel R est un groupe alkyle inférieur, et hydrolyse.

7. Procédé conforme à la revendication 5, dans lequel le produit est le composé de formule (I) dans lequel $R^2$ est un groupe méthyle.

8. Procédé conforme à la revendication 5, dans lequel le produit est la 7 - (1 - hydroxycarbonyléthyl)-oxy - 3 - phényl - 4H - 1 - benzopyran - 4 - one.

9. Procédé conforme à la revendication 5, dans lequel le produit est la 7 - (1 - hydroxycarbonyléthyl)-oxy - 3 - (4 - hydroxyphényl) - 4H - 1 - benzopyran - 4 - one.